# FASCICULE DE BREVET EUROPEEN

(11) **EP 3 352 684 B1**
(45) Date de publication et mention de la délivrance du brevet: **04.09.2024**
(21) Numéro de dépôt: 16775543.8
(22) Date de dépôt: 21.09.2016
(51) Int. Cl.: A61B 17/12, A61B 17/24, A61F 13/15

(54) **SYSTÈME POUR LE TRAITEMENT D'UNE ÉPISTAXIS**
SYSTEM ZUR BEHANDLUNG VON EPISTAXIS
SYSTEM FOR TREATING AN EPISTAXIS

(30) Priorité: 21.09.2015 FR 1558872; 11.01.2016 FR 1650205
(43) Date de publication de la demande: 01.08.2018
(73) Titulaire: Vygon, 95440 Ecouen (FR)
(72) Inventeur: LESTOQUOY, Patrick, 59551 Attiches (FR)
(74) Mandataire: Regimbeau
(86) Numéro de dépôt international: PCT/EP2016/072419
(87) Numéro de publication internationale: WO 2017/050823

(56) Documents cités:
- WO-A1-2005/053589
- US-A1- 2014 276 627
- US-B1- 6 517 509

## Description

### DOMAINE DE L'INVENTION

L'invention concerne le domaine des dispositifs médicaux pour contrôler un écoulement nasal voire une hémorragie des fosses nasales (ou épistaxis).

### ARRIERE-PLAN TECHNOLOGIQUE

Une épistaxis est un écoulement sanglant provenant des fosses nasales. Ce saignement est très fréquent et la plupart du temps bénin. Dans certains cas cependant, il peut devenir, par son abondance, sa répétition ou la fragilité du terrain une véritable urgence médico-chirurgicale.

On a donc proposé des systèmes adaptés pour absorber le sang et effectuer une vasoconstriction par compression contre les parois des fosses nasales sont introduits dans la cavité nasale du patient, afin de traiter ces épistaxis.

Par exemple, il existe des mèches possédant des propriétés d'hémostase et réalisées dans un matériau expansible configuré pour remplir la cavité nasale. Typiquement, les mèches peuvent être réalisées en cellulose régénéré oxydée, et donc être hémostatiques per se, ou dans un matériau expansible non hémostatique, tel que de l'alcool polyvinylique. Toutefois, de telles mèches sont traumatisantes à l'introduction et au retrait, malgré leur éventuelle ré-humidification avant retrait. Par ailleurs, un risque important d'hémorragie secondaire dû à l'arrachement non maîtrisé de la mèche persiste au point de jonction de l'hémostase.

On connaît également des systèmes de ballon gonflable comprenant une première configuration, dans laquelle le ballon est dégonflé afin d'être inséré dans la cavité nasale, et une deuxième configuration dans laquelle le ballon est gonflé afin de venir appliquer une pression contre la fosse nasale et arrêter l'hémorragie. Cependant, ces systèmes sont particulièrement inconfortables pour le patient en raison de la pression importante appliquée par le ballon afin d'arrêter l'hémorragie, sachant que cette pression est souvent plus importante que nécessaire pour garantir que le ballon s'adapte à la physionomie de la cavité nasale du patient. Qui plus est, ces systèmes ne présentent pas d'effet d'hémostase rapide. De plus, ces systèmes comprenant un ballon gonflable sont souples et sont donc difficiles à introduire et à positionner avec précision dans une cavité nasale d'un patient.

De plus, on connaît du document US 6,517,509 un système de traitement pour épistaxis qui comprend une mèche expansible et une gorge d'introduction de ladite mèche dans la cavité nasale. Cependant, un tel dispositif ne permet ni de contrôler la pression exercée sur les parois de la cavité nasale, ni d'éviter d'hémorragie secondaire au moment du retrait de la mèche. On remarque en outre que l'introduction de la mèche dans la cavité nasale est difficile dans la mesure où elle n'est pas guidée par la gorge d'introduction.

Par ailleurs, on connaît du document WO 2005/053589 un système de retrait de fluide organique d'un corps qui permet de facilement récupérer une mèche hémostatique. Cependant, un tel dispositif ne permet ni de contrôler la pression exercée sur les parois de la zone du corps à éponger, ni de retirer la mèche de manière maîtrisée et atraumatique. Ce système n'est en outre pas adapté à l'introduction d'une mèche dans une cavité nasale.

Enfin, on connaît du document US 2014/0276627 un système de succion nasale de fluide qui permet d'éponger un conduit nasal à l'aide d'une mèche expansible. Toutefois, un tel dispositif ne permet pas de contrôler la pression exercée sur les parois du conduit ni de retirer de façon atraumatique la mèche de la cavité.

### RESUME DE L'INVENTION

Un objectif de l'invention est donc de proposer un système pour le traitement d'une épistaxis qui permette d'arrêter une hémorragie nasale sans risque d'hémorragie secondaire au point de jonction de l'hémostase en appliquant une pression contrôlée sur les parois d'une cavité nasale d'un patient et qui soit en outre simple à mettre en oeuvre tout en étant plus ergonomique et moins traumatisant pour le patient que les systèmes de l'art antérieur.

Pour cela, l'invention propose un système pour le traitement d'une épistaxis comprenant :
- un organe compressible à mémoire de forme présentant une configuration comprimée, dans laquelle l'organe compressible est comprimé et est configuré pour être introduit dans une cavité nasale d'un patient, et une configuration expansée, dans laquelle l'organe compressible est déployé et est configuré pour venir en contact avec des parois de la cavité nasale du patient,
- une enveloppe configurée pour envelopper l'organe compressible, et
- un dispositif de compression de l'organe compressible dans l'enveloppe, ledit dispositif de compression étant configuré pour amener l'organe compressible dans sa configuration comprimée ou déployée.

Certaines caractéristiques préférées mais non limitatives du système décrit ci-dessus sont les suivantes, prises individuellement ou en combinaison :
- le dispositif de compression comprend un tube d'implantation, configuré pour être introduit dans la cavité nasale d'un patient, ledit tube d'implantation présentant une extrémité proximale, une extrémité distale et un passage central traversant s'étendant entre l'extrémité proximale et l'extrémité distale, et ledit organe compressible étant configuré pour être logé dans le passage central du tube d'implantation en configuration comprimée, et une attache configurée pour être fixée sur l'enveloppe et faire saillie de l'extrémité proximale du tube d'implantation lorsque l'organe compressible est logé dans le passage central, afin de faciliter l'introduction de l'organe compressible et de l'enveloppe dans le tube d'implantation lorsque l'organe compressible est en configuration expansée,
- l'enveloppe comprend un filet ou un film.
- l'enveloppe est configurée pour enfermer hermétiquement l'organe compressible de manière à former une enceinte étanche, et le dispositif de compression est configuré pour diminuer une pression dans l'ensemble formé de l'organe compressible enfermé dans l'enveloppe de manière à amener l'organe compressible dans sa configuration comprimée,
- l'enveloppe comprend un film souple,
- l'enveloppe comprend un film polyéthylène et/ou un film polyuréthane, ladite enveloppe pouvant présenter une épaisseur comprise entre quelques microns et une centaine de microns,
- le dispositif de compression comprend une seringue ou un organe de mise sous vide partiel,
- l'organe compressible présente une extrémité proximale à proximité de laquelle est fixé le dispositif de compression et une extrémité distale opposée à l'extrémité proximale, un évidement central étant formé entre l'extrémité proximale et l'extrémité distale de l'organe compressible et le système comprenant en outre un tube, logé dans l'évidement central, I
- le tube est configuré pour être mis en communication fluidique avec le dispositif de compression afin de réduire la pression dans l'organe compressible, des orifices traversants étant formés dans le tube entre l'extrémité proximale et l'extrémité distale de l'organe compressible afin de permettre une compression et une expansion progressives de l'organe compressible sur toute sa longueur,
- le système comprend en outre un verrou comprenant un premier organe de fixation, fixé sur une extrémité proximale du tube et un deuxième organe de fixation complémentaire fixé sur le dispositif de compression,
- le tube fait saillie de l'organe compressible et comprend une ailette, configurée pour être fixée sur une zone du corps d'un patient, et/ou
- le système comprend en outre un voile présentant des propriétés hémostatiques recouvrant l'enveloppe, ledit voile pouvant comprendre un voile en nontissé ayant un pH acide afin de lui conférer des propriétés hémostatiques.

Selon un deuxième aspect, l'invention propose un ensemble pour le traitement d'une épistaxis, comprenant :
- un organe compressible présentant une configuration comprimée, dans laquelle l'organe compressible est comprimé et est configuré pour être introduit dans une cavité nasale d'un patient, et une configuration expansée, dans laquelle l'organe compressible est déployé et est configuré pour venir en contact avec des parois de la cavité nasale du patient, et
- une enveloppe, ladite enveloppe entourant l'organe compressible et étant fixée sur ledit organe compressible de manière à former une enceinte étanche.

Cet ensemble est configuré pour être mis en oeuvre dans un système pour le traitement d'une épistaxis comme décrit ci-dessus.

Selon un troisième aspect, l'invention propose également un propose un système pour le traitement d'une épistaxis comprenant :
- un tube d'implantation, configuré pour être introduit dans une cavité nasale d'un patient, ledit tube d'implantation présentant une extrémité proximale, une extrémité distale et un passage central traversant s'étendant entre l'extrémité proximale et l'extrémité distale,
- un organe compressible ayant des propriétés hémostatiques, ledit organe compressible présentant une configuration comprimée, dans laquelle l'organe compressible est comprimé et est configuré pour être logé dans le passage central du tube d'implantation, et une configuration expansée, dans laquelle l'organe compressible est déployé et est configuré pour venir en contact avec des parois de la cavité nasale du patient,
- une enveloppe configurée pour envelopper l'organe compressible, et
- une attache configurée pour être fixée sur l'enveloppe et faire saillie de l'extrémité proximale du tube d'implantation lorsque l'organe compressible est logé dans le passage central, afin de faciliter l'introduction de l'organe compressible et de l'enveloppe dans le tube d'implantation lorsque l'organe compressible est en configuration expansée.

Certaines caractéristiques préférées mais non limitatives du système pour le traitement d'une épistaxis selon le troisième aspect de l'invention sont les suivantes, prises individuellement ou en combinaison :
- l'attache est configurée pour s'étendre dans le passage central et faire saillie de l'extrémité proximale du tube d'implantation lorsque l'organe compressible et l'enveloppe sont au moins partiellement logés dans le tube d'implantation,
- l'attache comprend au moins un brin ou un instrument comportant un crochet ou une pince,
- l'enveloppe comprend un filet,
- le filet comprend un matériau présentant des propriétés anticoagulation,
- l'organe compressible comprend une mousse, par exemple une mousse en cellulose régénéré oxydée présentant des propriétés hémostatiques, une mousse réalisée dans un matériau ne présentant pas de propriétés hémostatiques enveloppée dans un voile réalisé dans une cellulose régénérée oxydée présentant des propriétés hémostatiques ou encore une mousse réalisée dans un matériau ne présentant pas de propriétés hémostatiques imbibée d'un fluide présentant des propriétés hémostatiques,
- l'enveloppe comprend un film,
- le système comprend en outre un voile présentant des propriétés hémostatiques recouvrant l'enveloppe, ledit voile pouvant comprendre un voile en nontissé ayant un pH acide afin de lui conférer des propriétés hémostatiques,
- l'organe compressible et le tube d'implantation présentent une forme globalement cylindrique, de préférence cylindrique de révolution,
- le système comprend en outre un cathéter et l'organe compressible présente un évidement central configuré pour recevoir à coulissement ledit cathéter,
- le cathéter comprend une extrémité proximale, configurée pour être mise en communication fluidique avec un réservoir de fluide, et une extrémité distale, opposée à l'extrémité proximale et dans laquelle est formé un orifice d'injection, ledit orifice d'injection étant en communication fluidique avec l'extrémité proximale, et/ou
- le système comprend en outre un mandrin configuré pour être logé dans le tube d'implantation afin de pousser l'organe compressible de l'extrémité distale dudit tube d'implantation et permettre ainsi le retrait du tube d'implantation.

Selon un quatrième aspect, l'invention propose également un ensemble pour le traitement d'une épistaxis comprenant :
- un organe compressible ayant des propriétés hémostatiques, ledit organe compressible présentant une configuration comprimée, dans laquelle l'organe compressible est comprimé et est configuré pour être logé dans le passage central du tube d'implantation d'un système selon le troisième aspect de l'invention, et une configuration expansée, dans laquelle l'organe compressible est déployé et est configuré pour venir en contact avec des parois de la cavité nasale du patient,
- une enveloppe configurée pour envelopper l'organe compressible, et
- une attache configurée pour être fixée sur l'enveloppe et faire saillie de l'extrémité proximale du tube d'implantation lorsque l'organe compressible est logé dans le passage central, afin de faciliter l'introduction de l'organe compressible et l'enveloppe dans le tube d'implantation lorsque l'organe compressible est en configuration expansée.

### BREVE DESCRIPTION DES DESSINS

D'autres caractéristiques, buts et avantages de la présente invention apparaîtront mieux à la lecture de la description détaillée qui va suivre, et au regard des dessins annexés donnés à titre d'exemples non limitatifs et sur lesquels :
La figure 1 est une vue en coupe longitudinale d'un exemple de réalisation d'un système de traitement d'une épistaxis conforme à un premier mode de réalisation de l'invention,
La figure 2 est une vue en perspective d'un exemple de réalisation d'un organe compressible enveloppé dans une enveloppe d'un système conforme au premier mode de réalisation de l'invention,
La figure 3 est une vue en perspective de l'organe compressible de la figure 2,
La figure 4 est une vue de côté d'un exemple de réalisation d'un cathéter pouvant être utilisé dans un système de traitement conforme au premier mode de réalisation de l'invention,
La figure 5 est une vue de côté d'un exemple de réalisation d'un mandrin pouvant être utilisé avec un système de traitement conforme au premier mode de réalisation de l'invention,
La figure 6 est une vue partielle de côté d'un premier exemple de réalisation d'un système de traitement d'une épistaxis conforme à un deuxième mode de réalisation de l'invention,
La figure 7 est une vue en coupe du système de traitement de la figure 6, selon l'axe A-A,
La figure 8 est une vue de côté d'un deuxième exemple de réalisation d'un système de traitement d'une épistaxis conforme au deuxième mode de réalisation de l'invention.

### DESCRIPTION DETAILLEE D'UN MODE DE REALISATION

Dans ce qui suit, « proximal » désignera une partie qui se situe à proximité de l'opérateur lorsque celui-ci utilise le système de l'invention.

Afin d'assurer l'arrêt d'une épistaxis dans une cavité nasale d'un patient, l'invention propose un système 1 pour le traitement d'une épistaxis comprenant :
- un organe compressible 20, 120 présentant une configuration comprimée, dans laquelle l'organe compressible 20, 120 est comprimé et est configuré pour être introduit dans une cavité nasale d'un patient, et une configuration expansée, dans laquelle l'organe compressible 20, 120 est déployé et est configuré pour venir en contact avec des parois de la cavité nasale du patient,
- une enveloppe 30, 130 configurée pour envelopper l'organe compressible 20, 120, et
- un dispositif de compression 10, 110 de l'organe compressible 20, 120 dans l'enveloppe 30, 130, ledit dispositif de compression 10, 110 étant configuré pour amener l'organe compressible 20, 120 dans sa configuration comprimée ou déployée.

L'organe compressible 20, 120 présente une forme adaptée pour s'ajuster, en configuration expansée, à la forme de la cavité nasale du patient. Par exemple, l'organe compressible 20, 120 peut être de forme sensiblement cylindrique (de section carrée, parallélépipédique, ovale, ronde, etc.), voire cylindrique de révolution, ou en variante présenter une section non-uniforme entre son extrémité distale 24, 124 et son extrémité proximale 22, 122. Typiquement l'organe compressible 20, 120 peut présenter une portion distale plus étroite et une portion proximale élargie.

En configuration expansée, l'organe compressible 20, 120 présente une périphérie ayant une largeur L moyenne comprise entre vingt et quarante millimètres. Par largeur L de périphérie, on comprendra ici la distance entre deux droites parallèles (ou « lignes d'appui ») qui sont tangentes à la courbe fermée formée par la périphérie du capteur en deux points distincts. Par exemple, pour un organe compressible 20, 120 20 cylindrique de révolution, la largeur L moyenne est égale au diamètre externe du cylindre.

L'organe compressible 20, 120 peut notamment être réalisé dans un matériau compressible à mémoire de forme tel qu'une mousse, pouvant notamment présenter une densité de 25 kg/m³.

Contrairement aux systèmes de l'art antérieur mettant en oeuvre un ballon gonflable, l'organe compressible 20, 120 de l'invention est en configuration expansée lorsqu'il est au repos. La pression appliquée par l'organe compressible 20, 120 est donc facile à contrôler et résulte du matériau choisi pour le réaliser.

L'enveloppe 30, 130 peut avoir la forme d'une feuille plane et être enveloppée autour de l'organe compressible 20, 120. En variante, l'enveloppe 30, 130 peut présenter la forme générale d'un manchon conformé afin de pouvoir être enfilé de manière ajustée sur l'organe compressible 20, 120. En particulier, les dimensions de l'enveloppe 30, 130 en forme de manchon peuvent être ajustées aux dimensions et à la forme de l'organe compressible 20, 120 afin de limiter la formation de plis, qui formeraient des surépaisseurs susceptibles de créer un inconfort pour le patient. Par exemple, pour un organe compressible 20, 120 de forme cylindrique de révolution en configuration expansée, l'enveloppe 30, 130 peut présenter une forme cylindrique de révolution dont le diamètre externe est sensiblement égal au diamètre externe de l'organe compressible 20.

Le dispositif de compression 10, 110 peut comprendre un organe 10 configuré pour comprimer, de l'extérieur, l'organe compressible afin de l'introduire et de le retirer aisément d'une cavité nasale d'un patient (premier mode de réalisation). Le dispositif de compression 10, 110 peut sinon comprendre un organe 110 configuré pour comprimer, de l'intérieur (par aspiration de l'air dans l'organe compressible 120), l'organe compressible 120 (deuxième mode de réalisation).

### Premier mode de réalisation : compression par l'extérieur de l'organe compressible

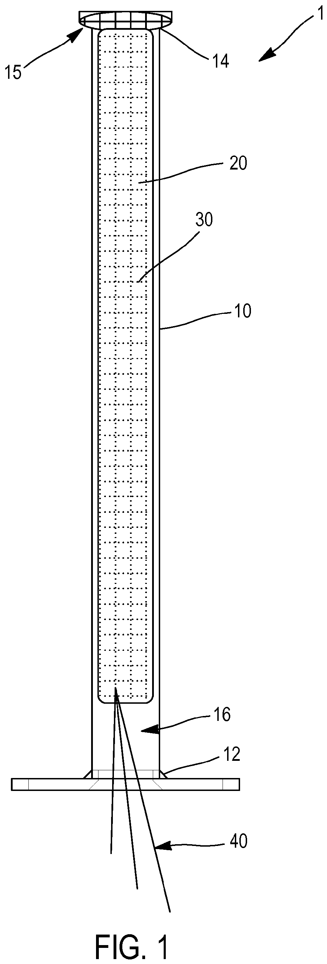

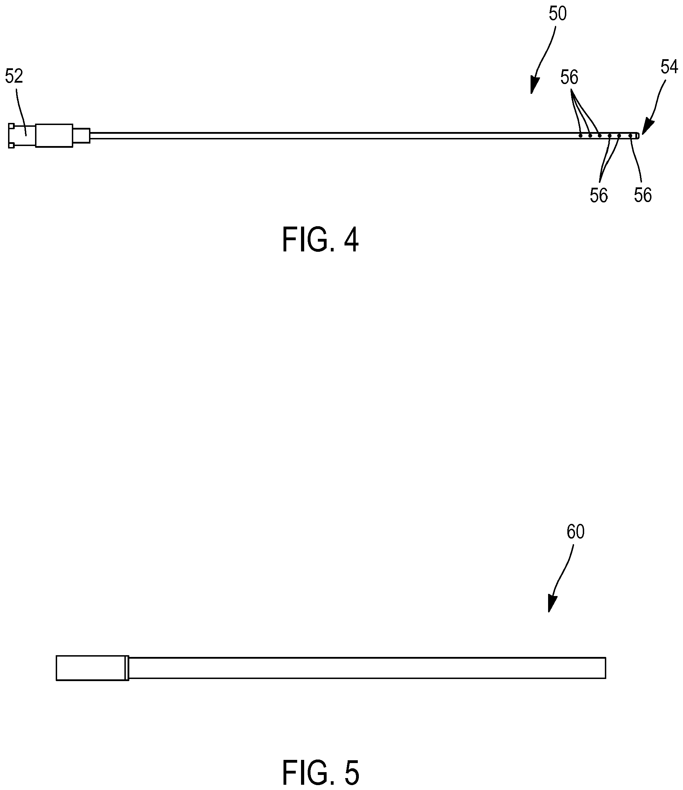

Dans ce premier mode de réalisation, illustré en référence aux figures 1 à 5, le dispositif de compression comprend un tube d'implantation 10, configuré pour être introduit dans la cavité nasale du patient, ledit tube d'implantation 10 présentant une extrémité proximale 12, une extrémité distale 14 et un passage central 16 traversant s'étendant entre l'extrémité proximale 12 et l'extrémité distale 14.
Par ailleurs, l'organe compressible 20 présente des propriétés hémostatiques. Enfin, le système comprend en outre une attache 40 configurée pour être fixée sur l'enveloppe 30 et faire saillie de l'extrémité proximale 12 du tube d'implantation 10 lorsque l'organe compressible 20 est logé dans le passage central 16, afin de faciliter l'introduction de l'organe compressible 20 et l'enveloppe 30 dans le tube d'implantation 10 lorsque l'organe compressible 20 est en configuration expansée.

L'organe compressible 20 peut notamment être réalisé dans un matériau compressible à mémoire de forme tel qu'une mousse, typiquement une mousse en cellulose régénéré oxydée présentant des propriétés hémostatiques, une mousse inerte - c'est-à-dire réalisée dans un matériau ne présentant pas de propriétés hémostatiques - enveloppée dans un voile réalisé dans une cellulose régénérée oxydée présentant des propriétés hémostatiques, ou une mousse inerte imbibée d'un fluide présentant des propriétés hémostatiques. Dans un exemple de réalisation, l'organe compressible 20 comprend une mousse de polyuréthane ayant une densité de 25 kg/m³ ayant des cellules ouvertes enveloppée dans un nontissé ayant un pH acide afin de conférer à l'organe compressible 20 des propriétés hémostatiques. Dans un autre exemple de réalisation, l'organe compressible 20 comprend une éponge de cellulose régénérée oxydée, qui présente des propriétés hémostatiques. Dans ce cas, afin de garantir que l'éponge 20 puisse adopter une configuration comprimée et une configuration expansée, l'éponge 20 en cellulose peut notamment être traitée à l'aide d'un agent plastifiant, tel que de sels de chlorure de magnésium.

L'enveloppe 30 a pour objectif de permettre, à l'aide de l'attache 40, la réintroduction de l'organe compressible 20 dans le tube d'implantation 10, lorsque l'organe compressible 20 est en configuration expansée.

Dans une première forme de réalisation, l'enveloppe 30 comprend un filet. Le filet 30 présente des mailles suffisamment grandes pour permettre la réalisation de l'hémostase dans la cavité nasale, mais suffisamment petites pour assurer le maintien de l'organe compressible 20 lors de son introduction dans la cavité nasale. Typiquement, le filet 30 peut présenter un maillage comprenant des mailles présentant une dimension comprise entre deux et cinq millimètres.

Le filet 30 peut être réalisé dans un matériau inerte.

En variante, le filet 30 peut être traité chimiquement afin de présenter des propriétés anticoagulantes. De la sorte, le traitement anticoagulant permet de limiter l'adhésion du filet 30 aux parois dans les zones discrètes de la paroi contre lesquelles le filet 30 est en contact, et donc de faciliter le retrait de l'organe compressible 20 de manière moins traumatisante pour le patient.

Dans une deuxième forme de réalisation, l'enveloppe 30 comprend un film souple.

De préférence, l'enveloppe 30 est réalisée dans un matériau non élastique. Par exemple, l'enveloppe 30 peut être réalisée en polyamide, en polyéthylène ou en polyuréthane.

L'attache 40 peut être formée intégralement avec l'enveloppe 30 ou rapportée et fixée sur l'enveloppe 30.

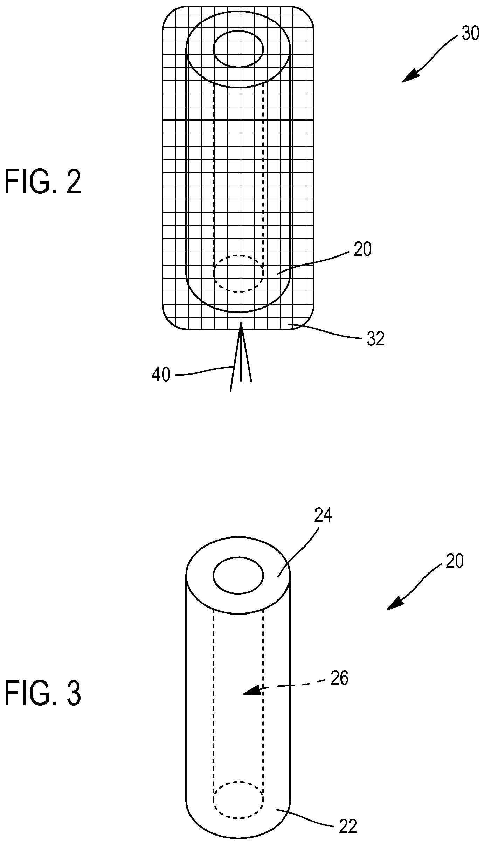

Dans un exemple de réalisation, comme visible sur la figure 2, l'attache 40 peut comprendre un ou plusieurs brins flexibles, fixés sur l'extrémité proximale 32 de l'enveloppe 30.

En variante, l'attache 40 peut comprendre un instrument rigide comprenant un crochet ou une pince fixé(e) à l'extrémité libre d'une tige rigide qui peut être réalisé en métal ou dans un matériau plastique. Dans le cas où l'enveloppe 30 comprend un filet, le crochet ou la pince de l'instrument peut alors être introduit(e) dans une ou plusieurs mailles du filet 30 alors que son extrémité proximale 32 reste accessible à un opérateur au niveau de l'extrémité proximale 12 du tube d'implantation 10.

Quelle que soit la forme de réalisation, la traction à l'aide de l'attache 40 sur l'enveloppe 30, qui entoure l'organe compressible 20, permet de détacher progressivement l'organe compressible 20 des parois de la cavité nasale et de l'amener progressivement en configuration comprimée de son extrémité proximale 22 vers son extrémité distale 24 grâce à sa réintroduction dans le tube d'implantation 10. Contrairement aux mèches conventionnelles, l'organe compressible 20 est donc décollé de proche en proche, et non détaché d'un seul bloc des parois de la cavité, réduisant ainsi le traumatisme subi par le patient. Une fois l'organe compressible 20 complètement introduit dans le tube d'implantation 10, celui-ci se trouve en configuration comprimée, dans laquelle son encombrement est réduit. L'extraction de l'organe compressible 20 est donc plus aisée pour un opérateur et plus confortable pour le patient que dans l'art antérieur, où les mèches sont extraites alors qu'elles sont imbibées de sang et ont un encombrement maximal.

Le tube d'implantation 10 présente une forme sensiblement cylindrique, par exemple cylindrique de révolution.

L'extrémité distale 14 du tube d'implantation 10 peut être évasée 15 afin de faciliter la réintroduction de l'organe compressible 20 en vue de sa compression progressive.

L'extrémité proximale 12 du tube d'implantation 10 peut comprendre une poignée équipée d'une garde.

Le diamètre interne du passage central 16 peut être compris entre six et douze millimètres.

Le cas échéant, le tube d'implantation 10 peut présenter, au niveau de sa périphérie, des graduations ou des repères afin de faciliter son positionnement dans la cavité nasale.

De manière optionnelle, le système 1 peut en outre comprendre un cathéter 50. Le cathéter 50 peut notamment permettre une réhumidification de l'organe compressible 20 et/ou l'injection d'un produit anesthésiant dans la cavité nasale avant le retrait de l'organe compressible 20 de la cavité nasale. A cet effet, l'organe compressible 20 peut notamment comprendre un évidement central 26 traversant, s'étendant entre l'extrémité proximale 22 et l'extrémité distale 24 de l'organe 20 et configuré pour recevoir à coulissement le cathéter 50. Dans le cas d'un organe compressible 20 cylindrique de révolution, l'évidement central 26 s'étend parallèlement à l'axe de révolution de l'organe compressible 20.

Le cathéter 50 peut comprendre une extrémité proximale 52, configurée pour être mise en communication fluidique avec un réservoir de fluide, et une extrémité distale 54, opposée à l'extrémité proximale 52 et destinée à être introduite dans l'évidement central 26 de l'organe compressible 20. Le cathéter 50 peut être mono-lumière et comprendre une lumière centrale traversante s'étendant entre son extrémité proximale 52 et son extrémité distale 54, ou en variante être multi-lumières et comprendre plusieurs lumières traversantes.

Dans une forme de réalisation, le cathéter 50 est mono-lumière et comprend un orifice d'injection 56 au niveau de son extrémité distale 54. Un tel cathéter 50 permet alors l'injection d'un produit anesthésiant préalablement à la mise en place de l'organe compressible 20 et/ou l'injection d'un produit pour assouplir l'organe compressible 20 préalablement à son retrait.

De manière alternative ou en complément, le cathéter 50 peut être mono-lumière et comprendre plusieurs orifices d'injection 56 formé à sa périphérie et répartis entre son extrémité distale 54 et son extrémité proximale 52 afin de permettre l'injection d'un fluide le long de l'évidement central 26 de l'organe compressible 20.

Optionnellement, le cathéter 50 peut être équipé de graduations ou de repères colorimétriques afin de permettre son positionnement précis dans l'organe compressible 20. Par exemple, le cathéter 50 peut comprendre des graduations ou des repères à intervalles réguliers, typiquement tous les centimètres, sur un tronçon adjacent à son extrémité distale 54 ou sur toute sa longueur.

Le cathéter 50 peut alors être utilisé afin d'injecter un produit présentant des propriétés hémostatiques dans l'organe compressible 20 préalablement à son implantation dans la cavité nasale, en particulier lorsque l'organe compressible 20 est réalisé dans un matériau n'ayant pas de telles propriétés. Dans ce cas, un cathéter 50 pourvu à sa périphérie d'une série d'orifices d'injection 56 répartis sur un tronçon adjacent à son extrémité distale 54 permet d'imbiber de manière efficace et contrôlée l'organe compressible 20.

Le système 1 pour le traitement d'une épistaxis conforme à ce premier mode de réalisation peut alors être mis en oeuvre comme suit.

Dans une première forme de réalisation, le système 1 pour le traitement d'une épistaxis peut être fourni en kit. Le système 1 doit alors être monté préalablement à son utilisation.

A cet effet, un organe compressible 20 est tout d'abord enveloppé ou introduit dans une enveloppe 30. A cette étape du montage, l'organe compressible 20 est en configuration déployée.

L'enveloppe 30 peut être pré-équipée d'une attache 40. En variante, une attache 40 peut être fixée sur l'enveloppe 30.

L'ensemble formé par l'organe compressible 20, l'enveloppe 30 et l'attache 40 est alors introduit dans un tube d'implantation 10. Par exemple, l'attache 40 peut être introduite dans le tube d'implantation 10 par son extrémité distale 14, puis l'organe compressible 20 - qui est alors en configuration déployée - est comprimé et introduit progressivement avec l'enveloppe 30 dans le tube d'implantation 10 par son extrémité distale 14 en appliquant un effort de traction sur l'attache 40 depuis l'extrémité proximale 12 du tube d'implantation 10. Une fois l'organe compressible 20 intégralement introduit dans le tube d'implantation 10, celui-ci se trouve alors en configuration comprimée.

En variante, l'organe compressible 20 et l'attache 40 peuvent également être introduits dans le tube d'implantation 10 par son extrémité proximale 12. Dans ce cas, l'organe compressible 20 et l'attache 40 peuvent être déplacés et amenés en position d'utilisation dans le tube d'implantation 10 à l'aide d'un mandrin 60.

Dans une deuxième forme de réalisation, le système 1 peut être partiellement préassemblé, l'organe compressible 20 étant enveloppé dans l'enveloppe 30 mais séparé du tube d'implantation 10. Dans ce cas, l'ensemble formé par l'organe compressible 20, l'enveloppe 30 et l'attache 40 est simplement introduit et amené en position d'utilisation dans un tube d'implantation 10, de manière analogue à la première forme de réalisation.

Dans une troisième forme de réalisation, le système 1 peut être préassemblé et prêt à l'emploi, l'organe compressible 20 étant enveloppé dans l'enveloppe 30 sur laquelle est fixée l'attache 40 et logé dans le tube d'implantation 10. Ici, l'organe compressible 20 est donc dans sa configuration comprimée.

Lorsque l'enveloppe 30 comprend un filet, la surface externe de l'organe compressible 20 présente de préférence des propriétés hémostatiques préalablement à son implantation dans la cavité nasale d'un patient. Pour cela, l'organe compressible 20 peut par exemple être réalisé dans un matériau présentant de telles propriétés (cas par exemple d'un organe compressible 20 comprenant de la cellulose régénérée oxydée). En variante, l'organe compressible 20 peut être imbibé à l'aide d'un fluide présentant ces propriétés hémostatiques. Dans ce dernier cas, une fois l'organe compressible 20 introduit dans le tube d'implantation 10, un cathéter 50 comprenant au moins un orifice d'injection peut être inséré dans l'évidement de l'organe compressible 20 à travers le tube d'implantation 10 afin d'injecter le fluide dans l'organe compressible 20. Selon une autre variante encore, l'organe compressible 20 peut être enveloppé dans un voile présentant des propriétés hémostatiques, tel qu'un voile en nontissé ayant un pH acide afin de lui conférer des propriétés hémostatiques du type Surgicel^{®}. Le voile présentant des propriétés hémostatiques est alors placé entre l'organe compressible 20 et le filet 30.

Lorsque l'enveloppe 30 comprend un film, un voile présentant des propriétés hémostatiques du type Surgicel^{®} est placé autour du film 30 enveloppant l'organe compressible 20 préalablement à leur introduction dans le tube d'implantation 10. En variante, le voile présentant des propriétés hémostatiques peut être placé autour du tube d'implantation, lors de la première étape décrite ci-après.

L'organe compressible 20 peut alors comprendre une mousse inerte.

Puis, au cours d'une première étape, le tube d'implantation 10 est amené et positionné dans la cavité nasale du patient, de sorte que son extrémité distale 14 soit placée au niveau de l'épistaxis. Le cas échéant, l'opérateur peut utiliser les éventuels repères ou graduations présents sur le tube d'implantation 10 afin de faciliter son positionnement dans la cavité nasale.

Au cours d'une deuxième étape, facultative, un agent hémostatique (sous forme de fluide) peut être injecté dans la cavité nasale du patient. Pour cela, lorsque l'organe compressible 20 comporte un évidement central 26, un cathéter 50 ayant un orifice d'injection distal 56 peut être introduit dans le passage central 16 du tube d'implantation 10 et dans l'évidement central 26 de l'organe compressible 20 et positionné de sorte que son orifice d'injection 56 se situe en regard de la cavité nasale. L'agent hémostatique peut alors être injecté. Le cathéter 50 peut ensuite être retiré du tube d'implantation 10.

En variante, un produit analgésique (anesthésiant local) peut également être injecté à l'aide d'une seringue préalablement à la première étape d'Introduction du tube d'implantation 10 dans la cavité nasale.

Selon une autre variante encore, le produit l'analgésique peut être injecté dans l'organe compressible 20, soit préalablement à son introduction dans le tube d'implantation 10, soit lorsque l'organe compressible 20 est comprimé dans ledit tube 10 (l'injection pouvant alors être effectuée à l'aide du cathéter 50).

Au cours d'une troisième étape, l'organe compressible 20 enveloppé dans l'enveloppe 30 et le cas échéant dans le voile présentant des propriétés hémostatiques peut être implanté dans la cavité nasale.

A cet effet, l'organe compressible 20 est amené dans une zone adjacente à l'extrémité distale 14 du tube d'implantation 10, comme visible sur la figure 1. Le cas échéant, dans cette position, l'organe compressible 20 commence à se déployer au niveau de l'extrémité évasée 15 du tube d'implantation 10. On notera que, dans une forme de réalisation, l'organe compressible 20 peut être placé dès le départ dans cette position, préalablement à l'étape d'introduction du tube d'implantation 10.

Un mandrin 60 peut alors être mis en oeuvre afin de maintenir l'organe compressible 20 en position pendant que, simultanément, le tube d'implantation 10 est progressivement extrait de la cavité nasale. Pour cela le mandrin 60 est introduit dans le tube d'implantation 10 par son extrémité proximale 12 et poussé jusqu'à ce qu'il vienne en contact avec l'organe compressible 20. On notera que, pendant cette manipulation, l'extrémité distale 14 de l'instrument de pose reste fixe par rapport à la cavité nasale, afin de garantir le positionnement de l'organe compressible 20. Un opérateur peut alors continuer de pousser sur le mandrin 60 et tirer simultanément sur le tube d'implantation 10, de manière à extraire l'organe compressible 20 de l'extrémité distale 14 et à retirer le tube d'implantation 10 de la cavité nasale. De la sorte, l'organe compressible 20 est extrait progressivement du tube d'implantation 10, la partie de l'organe compressible 20 faisant saillie de l'extrémité distale 14 du tube d'implantation 10 se déployant progressivement dans la cavité nasale.

Lorsque l'intégralité de l'organe compressible 20 est extraite du tube d'implantation 10, l'organe compressible 20, entouré de l'enveloppe 30 et le cas échéant du voile présentant des propriétés hémostatiques, se trouve alors positionné avec précision dans la cavité nasale et en configuration expansée. En effet, la position de l'organe compressible 20 est déterminée par la position de l'extrémité distale 14 du tube d'implantation 10 au début de la troisième étape, qui peut être précisément ajustée sans risquer de blesser le patient.

On notera que cette expansion progressive réduit en outre le traumatisme subit par le patient, l'organe compressible 20 n'étant à aucun moment déplacé.

Par ailleurs, en adoptant sa configuration expansée, l'organe compressible 20, qui est enveloppé dans l'enveloppe 30 et le cas échéant le voile présentant des propriétés hémostatiques, vient en contact avec les parois de la cavité nasale.

Dans une variante de réalisation, le mandrin 60 peut comprendre un orifice central afin de permettre à l'opérateur de laisser un cathéter 50 en place, dans l'organe compressible 20, malgré le retrait du tube d'implantation 10.

Le mandrin 60 et le tube d'implantation 10 peuvent alors être retirés de la cavité nasale.

Le cas échéant, lorsque l'attache 40 est amovible, celle-ci peut être détachée de l'enveloppe 30 afin de ne pas gêner le patient.

En variante, l'attache 40 peut être laissée en place.

Un phénomène d'hémostase peut alors avoir lieu, grâce aux propriétés hémostatiques de l'organe compressible 20 ou en variante du voile hémostatique qui l'entoure.

A la fin du phénomène d'hémostase, l'organe compressible 20 et l'enveloppe 30 peuvent alors être retirés.

Pour cela, au cours d'une quatrième étape, le tube d'implantation 10 peut être réintroduit dans la cavité nasale jusqu'à venir en butée contre l'extrémité proximale 22 de l'organe compressible 20. Le cas échéant, l'attache 40 est fixée sur l'enveloppe 30.

L'attache 40 est par ailleurs positionnée de manière à s'étendre dans le passage central 16 du tube d'implantation 10 en faisant saillie de son extrémité proximale 12.

Au cours d'une cinquième étape, optionnelle, lorsque l'enveloppe 30 comprend un filet, l'organe compressible 20 peut être ré-humidifié afin de l'assouplir en vue de son retrait. Par exemple, un cathéter 50 peut être introduit dans le passage central 16 du tube d'implantation 10 et dans l'évidement central 26 de l'organe compressible 20 afin d'injecter un fluide, tel que du sérum physiologique. Le cas échéant, l'opérateur peut utiliser les éventuels repères ou graduations présents sur le cathéter 50 afin de faciliter et d'améliorer son positionnement dans l'organe compressible 20.

En variante, l'étape de ré-humidification peut être réalisée préalablement à l'étape de mise en place du tube d'implantation 10, par exemple à l'aide d'une seringue.

Lorsque l'enveloppe 30 comprend un film, la ré-humidification de l'organe compressible 20 peut être optionnelle dans la mesure où ledit organe compressible 20 n'est pas contaminé, grâce au film qui forme une interface avec la cavité nasale du patient. L'organe compressible préserve donc ses propriétés de compression.

Au cours d'une sixième étape, le tube d'implantation 10 peut être avancé progressivement de manière à recevoir l'organe compressible 20 et l'enveloppe 30 dans son extrémité distale 14. Simultanément, un opérateur tire sur l'attache 40, afin d'éviter que l'organe compressible 20 ne bouge pendant la manipulation. De la sorte, l'organe compressible 20 est progressivement réintroduit et comprimé dans le tube d'implantation 10 et se décolle de proche en proche des parois de la cavité nasale, limitant ainsi fortement les risques d'hémorragie secondaire.

L'enveloppe 30, le tube d'implantation 10 et l'attache 40 permettent ainsi d'éviter un décollement soudain et complet sur toute la longueur de l'organe compressible 20, qui serait extrêmement douloureux pour le patient.

Le cas échéant, l'utilisation d'une enveloppe 30 comprenant un filet présentant des propriétés anticoagulantes permet de faciliter le décollement de l'organe compressible 20 en interrompant la continuité de la surface de l'organe compressible 20 susceptible d'adhérer aux parois de la cavité nasale, sans pour autant empêcher les phénomènes d'hémostase en raison de la faible surface de contact entre le filet 30 et les parois de la cavité.

Lorsque l'enveloppe comprend un film 30 entouré d'un voile présentant des propriétés hémostatiques, seuls le film 30 et l'organe compressible 20 sont réintroduits dans le tube d'implantation 10, le voile restant en place dans la cavité nasale. Le décollement du film 30 et de l'organe compressible 20 est alors facilité dans la mesure où la séparation se réalise au-delà de l'hémostase. Le voile présentant des propriétés hémostatiques peut ensuite être retiré séparément et de manière non traumatisante, par exemple à l'aide d'une pince, dans la mesure où celui-ci est sensiblement altéré suite à l'hémostase.

Le cas échéant, la réintroduction de l'organe compressible 20 dans le tube d'implantation 10 est également facilitée par la forme évasée 15 de l'extrémité distale 14 du tube d'implantation 10.

Lorsque l'intégralité de l'organe compressible 20 (emmanché dans l'enveloppe 30) est réinsérée dans le tube d'implantation 10, l'organe compressible 20 est alors en configuration comprimée.

Au cours d'une septième étape, le tube d'implantation 10 peut alors être retiré.

### Deuxième mode de réalisation : compression par l'intérieur de l'organe compressible

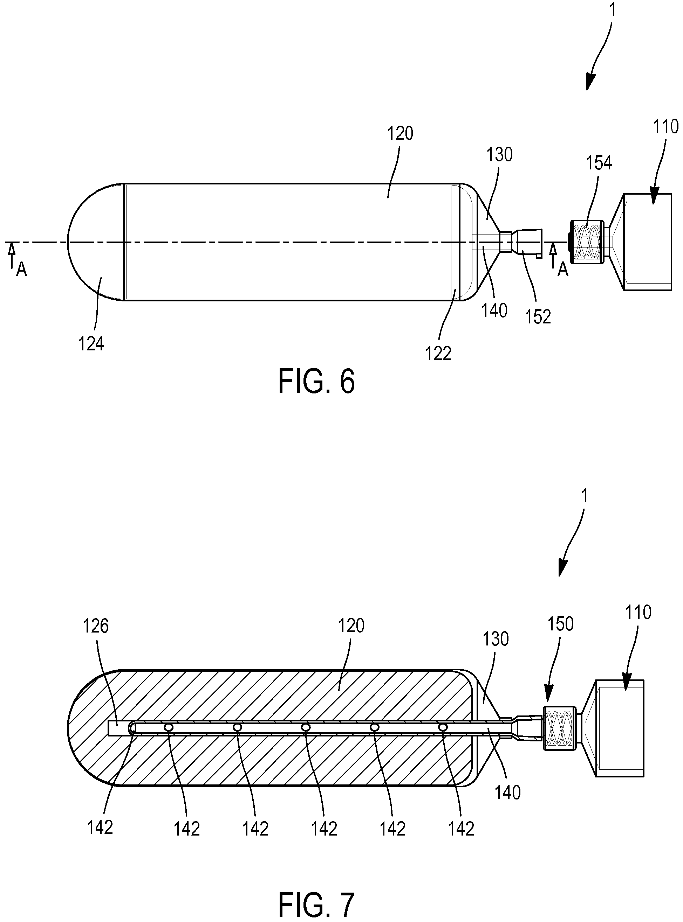

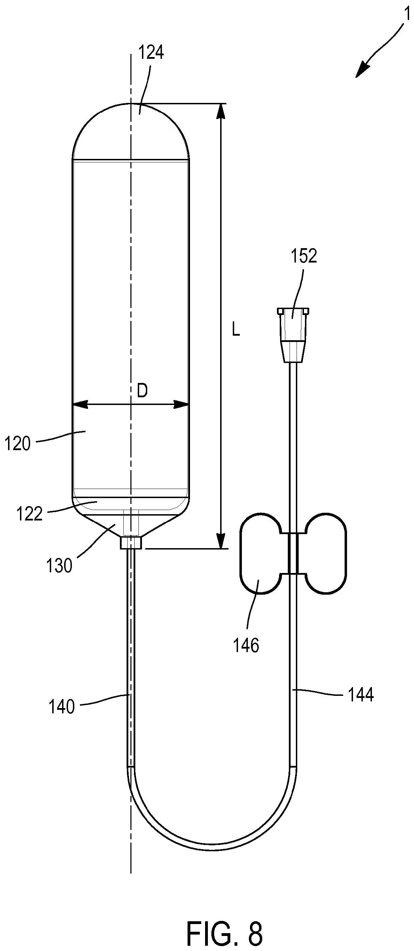

Dans ce deuxième mode de réalisation, illustré en référence aux figures 6 à 8, le dispositif de compression 110 est configuré pour aspirer de l'air dans l'organe compressible 120. A cet effet, le dispositif de compression 110 peut notamment comprendre un organe de mise sous vide partiel, capable par exemple d'atteindre une dépression dans l'organe compressible 120 de l'ordre de 450 mbar. Par ailleurs, l'enveloppe 130 est configurée pour enfermer de manière étanche l'organe compressible 120 afin de permettre cette mise sous vide partiel.

Le dispositif de compression 110 peut notamment comprendre une seringue, par exemple une seringue de 50 mL lorsque l'organe compressible 120 présente une longueur L de 10 cm pour un diamètre D de 2.8 à 2.9 cm.

En variante, le dispositif de compression 110 pourrait comprendre un système 1 de vide partiel conventionnellement utilisé dans les hôpitaux.

L'enveloppe 130, qui est ici étanche, doit être capable de résister à la différence de pression (par rapport à la cavité nasale, qui est à pression atmosphérique) créée par le dispositif de compression 110 lors de la mise sous vide partiel.

Pour cela, l'enveloppe 130 peut notamment comprendre un film souple qui peut être extensible ou non-extensible. L'enveloppe 130 peut par exemple comprendre un film polymère, tel qu'un film en polyéthylène, en polyamide ou en polyuréthane. L'enveloppe 130 peut présenter une épaisseur comprise entre quelques microns et une centaine de microns.

Contrairement aux systèmes de l'art antérieur utilisant un ballon gonflable dont la mise sous pression est difficile à contrôler, risquant ainsi de traumatiser le patient en appliquant des efforts trop importants sur les parois de sa cavité nasale, le système de l'invention propose donc d'appliquer une dépression à l'organe 120 permettant le phénomène d'hémostase lors de l'introduction ou de l'extraction du système et d'appliquer une pression contre les parois de la cavité nasale du patient en laissant simplement l'organe prendre de lui-même une configuration stable proche de sa configuration de repos.

De plus, lors de l'introduction de l'organe compressible 120 dans une cavité nasale, l'organe 120 est en configuration comprimée et est donc rigide en comparaison avec un ballon gonflable (qui est, au moment de son introduction, dégonflé et donc souple). La manipulation du système 1 est donc facilitée et plus précise.

Comme indiqué plus haut, l'organe compressible 120 peut comprendre une mousse. La mousse étant enfermée dans une enveloppe 130 hermétique, il n'est pas nécessaire qu'elle présente des propriétés hémostatiques. La mousse peut donc être inerte et être réalisée dans un matériau ne présentant pas de propriétés hémostatiques.

En revanche, l'enveloppe 130 peut elle-même être enfermée dans un voile présentant de plus des propriétés hémostatiques si l'on souhaite conférer au système 1 des propriétés hémostatiques, dans la mesure où ce voile est configuré pour entrer en contact avec les parois d'une cavité nasale. Un tel voile peut notamment être réalisé dans une cellulose régénérée oxydée présentant des propriétés hémostatiques. Le voile peut alors comprendre un voile en nontissé ayant un pH acide du type Surgicel^{®}. On notera que ce voile est cependant optionnel dans la mesure où l'effort de compression appliqué par l'organe compressible 120 lorsque celui-ci prend sa configuration expansée permet, à elle-seule, de réaliser une première hémostase.

Le cas échéant, l'enveloppe 130 peut être lubrifiée afin de faciliter l'introduction et le retrait du système 1 dans une cavité nasale d'un patient.

L'organe compressible 120 présente une extrémité distale 124, configurée pour être placée au fond d'une cavité nasale d'un patient, et une extrémité proximale 122 opposée à l'extrémité distale 124 au niveau de laquelle vient se connecter le dispositif de compression 110.

Afin de faciliter l'introduction de l'organe compressible 120 dans une cavité nasale d'un patient le système 1 peut en outre comprendre un tube 140 ou cathéter, inséré dans l'organe compressible 120 en vue de rigidifier celle-ci. A cet effet, un évidement 126 central peut être formé entre l'extrémité proximale 122 et l'extrémité distale 124 de l'organe compressible 120. Le tube 140 est alors logé dans cet évidement 126 central. L'ensemble formé par l'organe compressible 120 et le tube 140 est alors rigide, en particulier lorsque l'organe compressible 120 est en configuration comprimée, ce qui facilite grandement son introduction dans une cavité nasale (notamment en comparaison avec les systèmes à ballon, qui sont souples).

Dans une forme de réalisation, le tube 140 est fixe dans l'organe compressible 120. Le cas échéant le tube 140 peut être non amovible.

En variante, le tube est creux et est en communication fluidique avec le dispositif de compression 110. Des orifices traversants 142 sont en outre être formés dans la paroi du tube 140 afin de permettre au dispositif de compression 110 d'aspirer de l'air présent dans l'organe compressible 120 via le tube 140 et ses orifices 142. Les orifices traversants 142 s'étendent de préférence sur une grande partie de la longueur L de l'organe compressible 120, entre son extrémité proximale 122 et son extrémité distale 124, ce qui permet d'homogénéiser l'évacuation ou l'introduction de l'air lorsque l'organe compressible 120 est amené en configuration comprimée ou expansée. De la sorte, la compression et l'expansion de l'organe compressible 120 peuvent être progressifs et homogènes sur toute la surface de l'organe compressible 120, réduisant ainsi le traumatisme pour le patient lors de son expansion (mise en place) et de sa compression (au retrait).

Le tube 140 peut présenter une longueur sensiblement égale à la longueur L de l'organe compressible 120 et faire saillie dudit organe compressible 120 au niveau de son extrémité proximale 122.

En variante, le tube 140 peut présenter une longueur plus importante que celle de l'organe compressible 120 et comprendre une partie 144 configurée pour être fixée sur une zone du corps du patient, lorsque l'organe compressible 120 et le tube 140 sont placés dans une cavité nasale dudit patient. On notera en effet que l'organe compressible 120 est configuré pour rester en place dans une cavité nasale d'un patient pendant environ 48h. Il est donc préférable que, pendant cette période, le patient ne risque pas d'enfoncer davantage l'organe compressible 120 et puisse, autant que faire se peut, être installé confortablement. La Demanderesse s'est donc aperçue qu'en prolongeant le tube 140 de manière à le fixer sur la joue ou le bord du nez (par exemple) du patient, le patient risquait moins de déplacer l'organe compressible 120 que lorsque le tube 140 faisait tout juste saillie de son nez.

Le cas échéant, la partie rallongée 144 du tube 140 qui fait saillie du nez peut être équipée d'une ailette 146, afin de faciliter la fixation de cette partie rallongée 144 sur le corps du patient.

Le système 1 peut en outre comprendre un verrou 150 configuré pour permettre la connexion du dispositif de compression 110 sur l'enveloppe 130 et permettre ainsi l'évacuation ou l'introduction d'air dans l'organe compressible 120. Pour cela, le verrou 150 peut notamment comporter un premier organe de fixation 152, connecté à l'enveloppe 130 ou au tube 140, et un deuxième organe de fixation 154 complémentaire, connecté au dispositif de compression 110.

Dans le cas où le système 1 comprend un tube 140, le premier organe de fixation 152 peut être fixé sur l'extrémité du tube 140 qui fait saillie de l'organe compressible 120. L'enveloppe 130 est alors scellée hermétiquement à proximité de l'extrémité proximale 122 de l'organe compressible 120, soit sur le tube 140, soit sur le premier organe de fixation 152, de manière à former une enceinte étanche. Par exemple, l'enveloppe 130 peut être scellée par collage.

Lorsque le tube 140 est rallongé de manière à faire saillie du nez du patient et à être fixé sur une zone de son corps, le premier organe de fixation 152 peut être fixé au bout de la partie rallongée 144 du tube 140, à distance de l'organe compressible 120. Cette configuration permet en outre de faciliter la manipulation du système 1 par un opérateur, celui-ci étant plus libre de ses mouvements lors de l'aspiration de l'air à l'aide du dispositif de compression 110. L'enveloppe 130 est alors scellée hermétiquement sur le tube 140, à la sortie de l'organe compressible 120, de manière à former une enceinte étanche (par exemple par collage).

Le premier et le deuxième organe de fixation 152, 154 peuvent être conventionnels et comprendre par exemple un système du type Luer ou Luer lock.

Le système 1 pour le traitement d'une épistaxis conforme à ce deuxième mode de réalisation peut alors être mis en oeuvre comme suit.

La mise en oeuvre de ce deuxième mode de réalisation va être détaillée pour un système 1 comprenant un voile hémostatique et un tube 140, logé dans un évidement 126 central de l'organe compressible 120 et en communication fluidique avec le dispositif de compression 110. On comprendra cependant que l'utilisation du voile hémostatique est optionnelle.

Dans une première forme de réalisation, le système 1 pour le traitement d'une épistaxis peut être fourni en kit. Le système 1 doit alors être monté préalablement à son utilisation.

A cet effet, un organe compressible 120 est tout d'abord enveloppé ou introduit dans une enveloppe 130. A cette étape du montage, l'organe compressible 120 est en configuration déployée. L'organe compressible 120 peut, par exemple, comprendre une mousse inerte d'une longueur L comprise entre 8 et 12 cm, typiquement de l'ordre de 10 à 11 cm, et ayant un diamètre D compris entre 2.5 et 3.0 cm typiquement de l'ordre de 2.8 à 2.9 cm.

Un tube 140 comportant des orifices traversants 142 est alors introduit dans l'évidement 126 central de l'organe compressible 120. Le tube 140 peut par exemple présenter un diamètre externe de l'ordre de 2 à 3 mm et entre 3 et 10 orifices traversants 142 répartis sur la partie de sa longueur qui est logée dans l'organe compressible 120. Le tube 140 est en outre équipé, au niveau de son extrémité libre, d'un organe de fixation 152.

L'enveloppe 130 peut comprendre un film en polyuréthane ayant une épaisseur de 50 microns.

L'enveloppe 130 est ensuite scellée, par exemple sur une portion du tube 140 qui fait saillie de l'organe compressible 120, par exemple par collage.

Un dispositif de compression 110, par exemple une seringue comprenant un organe de fixation 154 complémentaire, est ensuite connectée à l'organe de fixation 152 du tube 140. Le dispositif de compression 110 peut alors réduire la pression (par rapport au milieu ambiant, qui est à pression atmosphérique) dans l'ensemble formé de l'organe compressible 120 enfermé dans l'enveloppe 130 en aspirant l'air contenu de ledit ensemble jusqu'à ce que l'organe compressible 120 atteigne sa configuration comprimée. Dans le cas d'une seringue, il suffit par exemple à un opérateur d'aspirer l'air en déplaçant le piston de la seringue.

Typiquement, pour un organe compressible 120 comprenant la mousse décrite ci-dessus, l'opérateur peut atteindre une dépression de l'ordre de 450 mbar. On notera qu'il est envisageable d'atteindre pression plus importante dans l'organe compressible : toutefois, la mousse serait moins comprimée, augmentant ainsi l'inconfort du patient lors de l'introduction de la mèche.

Puis l'ensemble formé de l'enveloppe 130, l'organe compressible 120 et le tube 140 peuvent être enveloppés dans un voile présentant des propriétés hémostatiques.

En variante, l'ensemble formé du tube 140 pré-introduit dans l'organe compressible 120 et enfermé de manière hermétique dans l'enveloppe 130 peut être préassemblé et fourni en kit à l'opérateur, le cas échéant préemballé dans le voile. Il suffit donc à l'opérateur de connecter le dispositif de compression 110, par exemple une seringue, au tube 140 afin de réduire la pression dans l'organe compressible 120 et d'amener l'organe compressible 120 dans sa configuration comprimée.

La seringue peut être fournie avec le kit qui est alors prêt à l'emploi, ou en variante être séparée du kit (cette variante étant plus facilement envisageable lorsque la connexion est normalisée, comme peut l'être une connexion Luer ou Luer lock).

Puis, au cours d'une première étape, l'ensemble (formé de l'organe compressible 120 en configuration comprimée, du tube 140, de l'enveloppe 130 et du voile), qui est fixé sur la seringue, est amené et positionné dans la cavité nasale du patient de sorte que l'extrémité distale 124 de l'organe compressible 120 soit placée au niveau de l'épistaxis. Cette introduction est facilitée par la présence du tube 140, qui permet de rigidifier l'ensemble sans augmenter pour autant l'encombrement de l'organe compressible 120.

L'organe compressible 120 (ainsi que le tube 140, l'enveloppe 130 et le voile) se trouve alors positionné avec précision dans la cavité nasale. En effet, la position de l'organe compressible 120 peut être aisément déterminée par la position de son extrémité distale 124, qui peut être ajustée sans risquer de blesser le patient dans la mesure où l'organe compressible 120 est en configuration comprimée et présente donc un encombrement réduit.

Au cours d'une deuxième étape, l'organe compressible 120 peut être déployé dans la cavité nasale. A cet effet, l'organe compressible 120 est amené de sa configuration comprimée vers sa configuration expansée, c'est-à-dire sa configuration stable et proche de sa position de repos (l'organe compressible 120 étant à mémoire de forme).

Dans le cas d'un dispositif de compression 110 comprenant une seringue, il suffit par exemple de déconnecter la seringue, l'organe compressible 120 reprenant progressivement sa forme grâce par aspiration passive d'air via les orifices traversants 142 et le tube 140. En variante, l'opérateur peut réinjecter l'air aspiré préalablement dans l'organe compressible 120 en poussant sur le piston de la seringue. De préférence, la réinjection est effectuée progressivement afin de ne pas traumatiser le patient.

Dans tous les cas, l'organe compressible 120 se déploie progressivement grâce aux orifices traversants 142 formés le long du tube 140, ce qui réduit le traumatisme subit par le patient, l'organe compressible 120 n'étant à aucun moment déplacé lors de son expansion. Par ailleurs, en adoptant sa configuration expansée, l'organe compressible 120, qui est enfermé dans l'enveloppe 130 et le cas échéant le voile présentant des propriétés hémostatiques, vient en contact avec les parois de la cavité nasale.

Le dispositif de compression 110 peut alors être retiré. Le cas échéant, lorsque le tube 140 comprend une partie rallongée, éventuellement équipée d'une ailette 146, ladite partie rallongée peut être fixée sur la peau du patient.

Un phénomène d'hémostase peut alors avoir lieu, grâce d'une part à l'effort de compression appliqué par l'organe compressible 120 sur les parois de la cavité nasale, et d'autre part aux propriétés hémostatiques du voile hémostatique qui entoure l'enveloppe 130 et l'organe compressible 120.

A la fin du phénomène d'hémostase, l'ensemble formé par l'organe compressible 120, l'enveloppe 130, le tube 140 et le voile peuvent alors être retirés.

Pour cela, au cours d'une troisième étape, le dispositif de compression 110, par exemple une seringue, peut être connecté sur l'ensemble. Par exemple, l'organe de fixation 154 de la seringue peut être connecté à l'organe de fixation 152 correspondant du tube 140.

Au cours d'une quatrième étape, la pression dans l'organe compressible 120 est réduite de manière à ramener l'organe compressible 120 dans sa configuration comprimée. Typiquement, pour un organe compressible 120 comprenant la mousse décrite ci-dessus, l'opérateur peut atteindre à nouveau la dépression de l'ordre de 450 mbar en déplaçant le piston de la seringue de manière à aspirer les fluides (notamment l'air) enfermé dans la mousse.

La mise sous vide partiel de l'organe compressible 120 est, ici encore, progressive. De plus, l'air est aspiré sur toute la longueur de l'organe compressible 120 grâce aux orifices traversants 142 formés dans le tube 140. De la sorte, l'organe compressible 120 est amené progressivement dans sa configuration comprimée, en se détachant lentement suivant une direction sensiblement perpendiculaire aux parois de la cavité nasale, par opposition à l'arrachement tangentiel et total qui était généralement obtenu avec les systèmes de traitement conventionnels.

Au cours d'une cinquième étape, lorsque l'organe compressible 120 (enfermé dans l'enveloppe 130 et le voile) a atteint sa configuration comprimée, il peut alors être extrait de la cavité nasale.

## Revendications

1. Système (1) pour le traitement d'une épistaxis comprenant :
- un organe compressible à mémoire de forme (20, 120) présentant une configuration comprimée, dans laquelle l'organe compressible (20, 120) est comprimé et est configuré pour être introduit dans une cavité nasale d'un patient, et une configuration expansée, dans laquelle l'organe compressible (20, 120) est déployé et est configuré pour venir en contact avec des parois de la cavité nasale du patient,
- une enveloppe (30, 130) configurée pour envelopper l'organe compressible (20, 120), ladite enveloppe comprenant un filet ou un film, et
- un dispositif de compression (10, 110) de l'organe compressible (20, 130) dans l'enveloppe (30, 130), ledit dispositif de compression (10, 110) étant configuré pour amener l'organe compressible (20) dans sa configuration comprimée ou déployée.

2. Système (1) selon la revendication 1 dans lequel :
- le dispositif de compression comprend un tube d'implantation (10), configuré pour être introduit dans la cavité nasale d'un patient, ledit tube d'implantation (10) présentant une extrémité proximale (12), une extrémité distale (14) et un passage central (16) traversant s'étendant entre l'extrémité proximale (12) et l'extrémité distale (14), et ledit organe compressible (20) étant configuré pour être logé dans le passage central (16) du tube d'implantation (10) en configuration comprimée, et
- une attache (40) configurée pour être fixée sur l'enveloppe (30) et faire saillie de l'extrémité proximale (12) du tube d'implantation (10) lorsque l'organe compressible (20) est logé dans le passage central (16), afin de faciliter l'introduction de l'organe compressible (20) et de l'enveloppe (30) dans le tube d'implantation (10) lorsque l'organe compressible (20) est en configuration expansée.

3. Système selon la revendication 1, dans lequel :
- l'enveloppe (130) est configurée pour enfermer hermétiquement l'organe compressible (120) de manière à former une enceinte étanche, et
- le dispositif de compression (110) est configuré pour diminuer une pression dans l'ensemble formé de l'organe compressible (120) enfermé dans l'enveloppe (130) de manière à amener l'organe compressible (120) dans sa configuration comprimée.

4. Système selon la revendication 3, dans lequel l'enveloppe (130) comprend un film souple.

5. Système selon la revendication 4, dans lequel l'enveloppe (130) comprend un film polyéthylène et/ou un film polyuréthane, ladite enveloppe (130) pouvant présenter une épaisseur comprise entre quelques microns et une centaine de microns.

6. Système selon l'une des revendications 3 à 5, dans lequel le dispositif de compression (110) comprend une seringue ou un organe de mise sous vide partiel.

7. Système (1) selon l'une des revendications 3 à 6, dans lequel l'organe compressible présente une extrémité proximale (122) à proximité de laquelle est fixé le dispositif de compression (110) et une extrémité distale (124) opposée à l'extrémité proximale (122), un évidement central (126) étant formé entre l'extrémité proximale (122) et l'extrémité distale (124) de l'organe compressible (120) et le système (1) comprenant en outre un tube (140), logé dans l'évidement central (126).

8. Système (1) selon la revendication 7, dans lequel le tube (140) est configuré pour être mis en communication fluidique avec le dispositif de compression (110) afin de réduire la pression dans l'organe compressible (120), des orifices traversants (142) étant formés dans le tube (140) entre l'extrémité proximale (122) et l'extrémité distale (124) de l'organe compressible (120) afin de permettre une compression et une expansion progressives de l'organe compressible (120) sur toute sa longueur.

9. Système selon l'une des revendications 7 ou 8, comprenant en outre un verrou (150) comprenant un premier organe de fixation (152), fixé sur une extrémité proximale (122) du tube (140) et un deuxième organe de fixation (154) complémentaire fixé sur le dispositif de compression (110).

10. Système selon l'une des revendications 7 à 9, dans lequel le tube (140) fait saillie de l'organe compressible (120) et comprend une ailette (146), configurée pour être fixée sur une zone du corps d'un patient.

11. Système (1) selon la revendication 1 à 10, dans lequel l'enveloppe (30, 130) a la forme d'une feuille plane ou d'un manchon.

12. Système selon l'une des revendications 1 à 11, comprenant en outre un voile présentant des propriétés hémostatiques recouvrant l'enveloppe (30, 130), ledit voile pouvant comprendre un voile en nontissé ayant un pH acide afin de lui conférer des propriétés hémostatiques.

13. Ensemble pour le traitement d'une épistaxis, comprenant :
- un organe compressible (120) présentant une configuration comprimée, dans laquelle l'organe compressible (20, 120) est comprimé et est configuré pour être introduit dans une cavité nasale d'un patient, et une configuration expansée, dans laquelle l'organe compressible (20, 120) est déployé et est configuré pour venir en contact avec des parois de la cavité nasale du patient, et
- une enveloppe (130), ladite enveloppe (130) entourant l'organe compressible (120) et étant fixée sur ledit organe compressible (120) de manière à former une enceinte étanche, et
- un dispositif de compression (10, 110) de l'organe compressible (20, 130) dans l'enveloppe (30, 130), ledit dispositif de compression (10, 110) étant configuré pour amener l'organe compressible (20) dans sa configuration comprimée ou déployée..

## Patentansprüche

1. System (1) zur Behandlung einer Epistaxis, das Folgendes umfasst:
- ein komprimierbares Element mit Formgedächtnis (20, 120), das eine komprimierte Konfiguration, in der das komprimierbare Element (20, 120) komprimiert und dazu konfiguriert ist, in eine Nasenhöhle eines Patienten eingeführt zu werden, und eine expandierte Konfiguration, in der das komprimierbare Element (20, 120) entfaltet und dazu konfiguriert ist, in Kontakt mit den Wänden der Nasenhöhle des Patienten zu kommen, aufweist,
- eine Hülle (30, 130), die dazu konfiguriert ist, das komprimierbare Element (20, 120) zu umhüllen, wobei die Hülle ein Netzgewebe oder eine Folie umfasst, und
- eine Kompressionsvorrichtung (10, 110) für das komprimierbare Element (20, 130) in der Hülle (30, 130), wobei die Kompressionsvorrichtung (10, 110) dazu konfiguriert ist, das komprimierbare Element (20) in seine komprimierte oder entfaltete Konfiguration zu bringen.

2. System (1) nach Anspruch 1, wobei:
- die Kompressionsvorrichtung einen Implantationsschlauch (10) umfasst, der dazu konfiguriert ist, in die Nasenhöhle eines Patienten eingeführt zu werden, wobei der Implantationsschlauch (10) ein proximales Ende (12), ein distales Ende (14) und einen zentralen Durchgang (16) aufweist, der sich zwischen dem proximalen Ende (12) und dem distalen Ende (14) erstreckt, und das komprimierbare Element (20) dazu konfiguriert ist, in der komprimierten Konfiguration im zentralen Durchgang (16) des Implantationsschlauchs (10) untergebracht zu werden, und
- ein Befestigungsmittel (40), das dazu konfiguriert ist, an der Hülle (30) befestigt zu werden und von dem proximalen Ende (12) des Implantationsschlauchs (10) hervorzustehen, wenn das komprimierbare Element (20) im zentralen Durchgang (16) untergebracht ist, um das Einführen des komprimierbaren Elements (20) und der Hülle (30) in den Implantationsschlauch (10) zu erleichtern, wenn sich das komprimierbare Element (20) in der expandierten Konfiguration befindet.

3. System nach Anspruch 1, wobei:
- die Hülle (130) dazu konfiguriert ist, das komprimierbare Element (120) hermetisch einzuschließen, um so ein dichtes Gehäuse zu bilden, und
- die Kompressionsvorrichtung (110) dazu konfiguriert ist, einen Druck in der Einheit zu verringern, die aus dem in der Hülle (130) eingeschlossenen komprimierbaren Element (120) gebildet wird, um das komprimierbare Element (120) in seine komprimierte Konfiguration zu bringen.

4. System nach Anspruch 3, wobei die Hülle (130) eine flexible Folie umfasst.

5. System nach Anspruch 4, wobei die Hülle (130) eine Polyethylenfolie und/oder eine Polyurethanfolie umfasst, wobei die Hülle (130) eine Dicke zwischen einigen Mikrometern und hundert Mikrometern aufweisen kann.

6. System nach einem der Ansprüche 3 bis 5, wobei die Kompressionsvorrichtung (110) eine Spritze oder ein Teilvakuumelement umfasst.

7. System (1) nach einem der Ansprüche 3 bis 6, wobei das komprimierbare Element ein proximales Ende (122), in dessen Nähe die Kompressionsvorrichtung (110) befestigt ist, und ein distales Ende (124) gegenüber dem proximalen Ende (122) aufweist, wobei eine zentrale Aussparung (126) zwischen dem proximalen Ende (122) und dem distalen Ende (124) des komprimierbaren Elements (120) gebildet ist und das System (1) ferner einen Schlauch (140) umfasst, der in der zentralen Aussparung (126) untergebracht ist.

8. System (1) nach Anspruch 7, wobei der Schlauch (140) dazu konfiguriert ist, in Fluidverbindung mit der Kompressionsvorrichtung (110) gebracht zu werden, um den Druck im komprimierbaren Element (120) zu reduzieren, wobei Durchgangsöffnungen (142) im Schlauch (140) zwischen dem proximalen Ende (122) und dem distalen Ende (124) des komprimierbaren Elements (120) gebildet sind, um eine allmähliche Kompression und eine allmähliche Expansion des komprimierbaren Elements (120) über seine gesamte Länge zu ermöglichen.

9. System nach einem der Ansprüche 7 oder 8, das ferner eine Verriegelung (150) umfasst, die ein erstes Befestigungselement (152), das an einem proximalen Ende (122) des Schlauchs (140) befestigt ist, und ein zweites komplementäres Befestigungselement (154) umfasst, das an der Kompressionsvorrichtung (110) befestigt ist.

10. System nach einem der Ansprüche 7 bis 9, wobei der Schlauch (140) von dem komprimierbaren Element (120) hervorsteht und einen Steg (146) umfasst, der dazu konfiguriert ist, an einem Körperbereich eines Patienten befestigt zu werden.

11. System (1) nach Anspruch 1 bis 10, wobei die Hülle (30, 130) die Form eines ebenen Blatts oder einer Hülse hat.

12. System nach einem der Ansprüche 1 bis 11, das ferner ein Gewebe umfasst, das hämostatische Eigenschaften aufweist und die Hülle (30, 130) bedeckt, wobei das Gewebe ein Vliesgewebe umfassen kann, das einen sauren pH-Wert hat, um ihm hämostatische Eigenschaften zu verleihen.

13. Einheit für die Behandlung einer Epistaxis, die Folgendes umfasst:
- ein komprimierbares Element (120), das eine komprimierte Konfiguration, in der das komprimierbare Element (20, 120) komprimiert und dazu konfiguriert ist, in eine Nasenhöhle eines Patienten eingeführt zu werden, und eine expandierte Konfiguration, in der das komprimierbare Element (20, 120) entfaltet und dazu konfiguriert ist, in Kontakt mit den Wänden der Nasenhöhle des Patienten zu kommen, aufweist, und
- eine Hülle (130), wobei die Hülle (130) das komprimierbare Element (120) umgibt und an dem komprimierbaren Element (120) befestigt ist, um ein dichtes Gehäuse zu bilden, und
- eine Kompressionsvorrichtung (10, 110) für das komprimierbare Element (20, 130) in der Hülle (30, 130), wobei die Kompressionsvorrichtung (10, 110) dazu konfiguriert ist, das komprimierbare Element (20) in seine komprimierte oder entfaltete Konfiguration zu bringen.

## Claims

1. A system (1) for the treatment of epistaxis comprising:
- a shape memory compressible member (20, 120) having a compressed configuration, in which the compressible member (20, 120) is compressed and is configured to be introduced into a nasal cavity of a patient, and an expanded configuration, in which the compressible member (20, 120) is expanded and is configured to contact walls of the nasal cavity of the patient,
- a sheath (30, 130) configured to envelop the compressible member (20, 120), said sheath comprising a net or a film, and
- a compression device (10, 110) of the compressible member (20, 130) within the sheath (30, 130), said compression device (10, 110) being configured to bring the compressible member (20) into its compressed or deployed configuration.

2. System (1) according to claim 1 wherein :
- the compression device comprises an implantation tube (10) configured to be inserted into the nasal cavity of a patient, said implantation tube (10) having a proximal end (12), a distal end (14) and a through central passageway (16) extending between the proximal end (12) and the distal end (14), and said compressible member (20) being configured to be received within the central passageway (16) of the implantation tube (10) in a compressed configuration, and
- a clip (40) configured to be fixed to the sheath (30) and to project from the proximal end (12) of the implantation tube (10) when the compressible member (20) is housed in the central passage (16), in order to facilitate the introduction of the compressible member (20) and the sheath (30) into the implantation tube (10) when the compressible member (20) is in the expanded configuration.

3. The system of claim 1, wherein :
- the sheath (130) is configured to hermetically enclose the compressible member (120) so as to form a sealed enclosure, and
- the compression device (110) is configured to reduce a pressure in the assembly formed by the compressible member (120) enclosed in the sheath (130) so as to bring the compressible member (120) into its compressed configuration.

4. A system as claimed in claim 3, wherein the sheath (130) comprises a flexible film.

5. System according to claim 4, wherein the sheath (130) comprises a polyethylene film and/or a polyurethane film, said sheath (130) being able to have a thickness of between a few microns and a hundred microns.

6. A system according to any one of claims 3 to 5, wherein the compression device (110) comprises a syringe or partial vacuum member.

7. A system (1) according to any of claims 3 to 6, wherein the compressible member has a proximal end (122) near which the compression device (110) is attached and a distal end (124) opposite the proximal end (122), a central recess (126) being formed between the proximal end (122) and the distal end (124) of the compressible member (120) and the system (1) further comprising a tube (140), housed in the central recess (126).

8. The system (1) of claim 7, wherein the tube (140) is configured to be placed in fluid communication with the compression device (110) to reduce the pressure in the compressible member (120), through-ports (142) being formed in the tube (140) between the proximal end (122) and the distal end (124) of the compressible member (120) to allow progressive compression and expansion of the compressible member (120) along its length.

9. System according to one of claims 7 or 8, further comprising a lock (150) comprising a first fixing member (152), fixed to a proximal end (122) of the tube (140) and a second complementary fixing member (154) fixed to the compression device (110).

10. A system according to any one of claims 7 to 9, wherein the tube (140) projects from the compressible member (120) and comprises a fin (146), configured to be secured to an area of a patient's body.

11. The system (1) of claim 1 to 10, wherein the sheath (30, 130) is in the form of a flat sheet or sleeve.

12. System according to one of claims 1 to 11, further comprising a veil with haemostatic properties covering the sheath (30, 130), said veil being able to comprise a non-woven veil with an acid pH in order to confer haemostatic properties.

13. Set for the treatment of epistaxis, comprising :
- a compressible member (120) having a compressed configuration, in which the compressible member (20, 120) is compressed and is configured to be introduced into a nasal cavity of a patient, and an expanded configuration, in which the compressible member (20, 120) is expanded and is configured to contact walls of the nasal cavity of the patient, and
- a sheath (130), said sheath (130) surrounding the compressible member (120) and being fixed to said compressible member (120) so as to form a sealed enclosure, and
- a compression device (10, 110) of the compressible member (20, 130) within the sheath (30, 130), said compression device (10, 110) being configured to bring the compressible member (20) into its compressed or deployed configuration.
